# EUROPEAN PATENT SPECIFICATION

(11) **EP 1 411 899 B1**
(45) Date of publication and mention of the grant of the patent: **11.03.2009**
(21) Application number: 02763407.0
(22) Date of filing: 01.08.2002
(51) Int. Cl.: A61K 9/14, A61K 9/20, A61K 31/192

(54) **TASTE MASKING COMPOSITION**
ZUSAMMENSETZUNG ZUR MASKIERUNG DES GESCHMACKS
COMPOSITION DE MASQUAGE DE GOUT

(30) Priority: 01.08.2001 US 309285 P
(43) Date of publication of application: 28.04.2004
(73) Proprietor: NOVARTIS AG, 4056 Basel (CH)
(72) Inventor: CORBO, Michael, Flemington, NJ 08822 (US); MIGTON, John, Clark, NJ 07066 (US); PATELL, Mahesh, Edison, NJ 08820 (US)
(74) Representative: Dietz, Jörg-Reimar
(86) International application number: PCT/US2002/024452
(87) International publication number: WO 2003/011227

(56) References cited:
- WO-A-00/25765
- WO-A-00/56266
- WO-A-00/76479
- WO-A-01/49276
- WO-A-97/16174
- WO-A-99/62498
- FR-A- 2 791 888
- FR-A- 2 791 889
- US-A- 4 755 386
- US-A- 4 808 411
- US-A- 5 945 405
- US-B1- 6 204 270

## Description

### FIELD OF THE INVENTION

The present invention relates to a composition containing a medicament, such as ibuprofen, which when released in the mouth or in contact with the throat mucosa, produces an unpleasant bitter taste and/or an unpleasant sensation in the throat Agents are disclosed which when incorporated in the composition mask these effects.

### BACKGROUND OF THE INVENTION

Swallowing tablets is a problem for many people particularly children and geriatric patients. The problem is exacerbated when the tablets are large. Chewable tablets alleviate this problem; however, additional problems arise when the tablets contain a medicament that is bitter tasting.

Various materials have been incorporated in chewable tablet formulations in order to mask the bitter taste of active components. One approach is to coat the bitter tasting active with a material that does not dissolve in the mouth. The coating must be capable of withstanding the high compressive force of tableting without rupturing. If the coating ruptures during tableting the bitter taste of the medicament will be evident.

Bitter taste is not the only problem encountered in formulating chewable tablets. Certain medicaments leave an unpleasant catch in the throat when they are orally ingested. Ibuprofen is an example of a drug that exhibits this unpleasant effect. Prior to the present invention, no taste-masking ingredient has been able to overcome this.

United States Patent 3,346,449 teaches the reaction of a d-methorphan acid addition salt, a bitter and unpleasant tasting material, with a polymeric material that is an acid carboxylvinyl polymer of acrylic acid copolymerized with from about 0.75% to about 2.0% by weight of polyallyl sucrose. Carbopol® 934 is the preferred acidic polymeric reactant. Patentees disclose that the reaction product of the d-methorphan acid addition salt with Carbopol® 934 does not have a residual bad taste and when embodied in conventional oral dosage forms possesses sustained release antitussive characteristics. In contradistinction thereto, the compositions of the present invention provide quick release of the active component not sustained release thereof. Moreover, compositions of the present invention are prepared by a simple blending of the composition ingredients. Unlike U.S. Patent 3,346,449, wherein d-methorphan acid addition salt is reacted with Carbopol ® 934 in water, the present invention does not employ a reaction product of ibuprofen and Carbopol ®.

U.S. patent 4,404,183 discloses amorphous nicardipine powder coated with a material that prevents disintegration and dissolution in the stomach. Patentees disclose that such effect can be obtained by adding a pH-depending agent, in viscosity-increasing agent or water-insoluble agent. Carbopol® (B.F. Goodrich Company) is mentioned as a suitable viscosity-increasing agent. Patentees, however, seek to formulate sustained release dosage forms as opposed to dosage forms that provide quick release of the active component. Moreover, patentees are not concerned with the formulation of a chewable tablet or taste masking a bitter drug contained therein.

U.S. patent, 4,837,111 discloses a dosage form for dispensing a drug for human therapy. Carbopol®-acidic carboxy polymers having a molecular weight of 450,000 to 4 million are indicated to be suitable osmopolymers. However, the invention of this patent is directed to an osmotic device. There is no appreciation whatsoever on the part of patentees of the use of Carbopol® for taste masking, much less for taste masking a bitter drug, more particularly a drug that causes throat catch. Moreover, patentees are not concerned with the production of a chewable tablet utilizing a formulation that is produced by a simple dry blending operation.

U.S. Patent 4,902,514 is directed to a dosage form for administering nilvadipine. This patent is distinguishable from the present invention on essentially the same grounds that the invention of U.S. Patent 4,837,111 was distinguished. In U.S. Patent 4,902,514, the drug is contained in a laminate, which in turn is encased within a coating layer. The dosage form produces sustained release of the active. There is no disclosure of chewable tablets, much less chewable tablets that provide quick release of drug contained therein. Consequently this patent does not deal with the problem of taste masking a drug that is bitter and/or causes throat catch.

U.S. Patent 4,649,043 discloses a drug delivery system for delivering a plurality of tiny pills in the gastro-intestinal tract. In the drug delivery device of this patent the drug is coated for sustained release, then dispersed in a hydrogel matrix. Numerous hydrophilic polymer materials, including hydrated polyethylene oxide (Polyox®) and Carbopol® acidic carboxypolymer are disclosed to be useful. U.S. Patent 4,649,043 is directed to a dosage form that provides sustained (rather than quick) release of the drug (Col.3, Lines 53-56, where patentees disclose that the drug delivery device of the invention "houses a multiplicity of tiny pills for the controlled delivery of drug over time"). Additionally, patentees are not concerned with the problem of producing a chewable tablet containing a drug that is bitter and/or causes throat catch or masking these undesirable effects

U.S. Patent 4,808,411 discloses compositions comprising from about 25% to about 90% erythromycin or a derivative thereof, and from about 10% to about 75% of a carbomer. It is asserted that such compositions provide palatable dosages of the antibiotic yet have pharmacokinetic properties substantially equivalent to that of commercially available tablets and capsules. Patentees also point out that erythromycin (particularly 6-O methyl erythromycin) has a bitter taste. Patentees disclose that the carbomers employed in the invention are acrylic acid polymers commercially available from B.F. Goodrich Company and others and having an average equivalent weight of 76 and a molecular weight of approximately 3 million. It is disclosed that they conform to the general formula [-CH₂-CH(COOH)-]ₙ wherein ₙ is from about 10,000 to about 60,000. The preferred material is disclosed to be carbomer 934P. Compositions are prepared by dispersing the erythromycin compound in a suitable organic solvent, such as ethanol or acetone, and dispersing the carbomer separately in ethanol. The two solutions are then mixed slowly to allow formation of "the desired reaction product" (Col.3, lines 49-50). Most of the organic solvent is then evaporated off and the solution is then diluted with water. The reaction product is recovered by filtration then dried. Patentees disclosed an alternative method of preparation wherein a slurry of a mixture of erythromycin, or erythromycin derivative, and carbomer is prepared by blending same in a limited amount of organic solvent. This is followed by evaporation and drying. Patentees point out that a reaction product results. The reaction product is believed to be held together by ionic attraction between the amine group of the erythromycin compound and the carbonyl group of the carbomer, and by the gel properties of the insoluble carbomer (Col.3, lines 58-63). More importantly, patentees assert that the formation of such reaction product "is important for both stability of the drug and palatability of the composition" (Col.3, lines 63-64). Patentees state that when ingested, the erythromycin compound is released from the complex slowly enough to avoid a significant perception of bitterness in the mouth (Col.4, lines 24-26). Patentees also disclose that the antibiotic/carbomer complexes of their invention can be employed in dry form or formed in the conventional or chewable tablets for oral administration. In contrast to the teaching of the invention of U.S. Patent 4,808,411 the present invention does not call for the production of a reaction product between an active and carbomer in order produce a complex that is subsequently formed into a chewable tablet for oral administration. Additionally, the present invention achieves taste masking of a bitter active and/or avoidance of throat catch caused by said active, while simultaneously producing a formulation and/or chewable tablet dosage form prepared therefrom, which provides quick release of the active. In contrast thereto, in U.S. Patent 4,808,411 when the bitter erythromycin compound is ingested it is released from the complex slowly enough to avoid a significant perception of bitterness in the mouth. It is clear that slow release, not quick release, of the bitter active ingredient is critical to the '411 invention.

U.S. Patent 5,552,152 discloses a chewable taste-masked tablet having controlled release characteristics. The tablet consists essentially of a microcapsule of about 100 microns to about 0.8 mm in diameter having a pharmaceutical core including crystalline and ibuprofen and a methacrylic acid copolymer coating having sufficient elasticity to withstand coating. The methacrylic acid copolymer can be a copolymer of polymethacrylic acid and acrylic acid esters. Patentees teach that the polymeric coating should provide for immediate release characteristics "i.e., rapid release of the active agents in the duodenum within a period of about one hour" (see Col.2, lines 55-57). Patentees state that when the microcapsules are formulated into chewable taste masked oral tablets or capsules, the formulations provide for immediate, rapid release in the stomach (Col. 2, lines 57-60). Thus, the invention of this patent is distinguishable from that of the present invention in that the present invention provides for substantial immediate release of the bitter active agent whereas the invention of this patent provides for delayed release. This is evident by the teaching of U.S. Patent 5,552,152 that the invention of such patent contemplates elastic microcapsules that do not release ibuprofen in the mouth when chewed (Col.2, lines 25-27) and by the disclosure that release of the actives occurs in either the duodenum or in the stomach and not in the mouth. Since release of ibuprofen occurs in either the duodenum or stomach, rather than in the mouth, the bitter taste in the mouth or throat catch caused by ibuprofen is not a problem confronted by patentees. Consequently, there is no teaching or even suggestion in this patent of a formulation that would solve the problem of the bitter taste or throat catch caused by chewing a tablet which release ibuprofen in the mouth.

European Patent Application Publication Number 0636365A1 discloses a freeze- dried pharmaceutical dosage form containing a porous matrix of a water-soluble or water-dispersible carrier material containing a coated pharmaceutical particle. The pharmaceutical granule is coated with a blend of a first polymer selected from the group consisting of cellulose acetate and a cellulose acetate butyrate and second polymer selected from the group consisting of polyvinyl pyrrolidone and hydroxypropyl cellulose. Patentees disclose that their invention provides a freeze-dried dosage form containing a pharmaceutical coated with the material that provides taste masking and protection against the leaching of the pharmaceutical into the solution of the carrier material during the freeze-drying process. Basically, the teaching of this application calls for coating a bad tasting active that is contained in a porous matrix. The bad tasting active is coated with two polymer materials. In Example 1 of the application, APAP coated with cellulose acetate and PVP is employed. In Comparative Example A, APAP coated with cellulose acetate and dibutyl sebecate is employed. Example 2 and Comparative Example B respectively employ coated and spray-dry APAP particles.

Although Polyox® has been employed in the prior art as an excipient in controlled release pharmaceuticals (U.S. Patents 4,649,043, 4,902,514, 4,837,111 and 4,404,183) its' use for taste masking has not been appreciated prior to the present invention.

WO-A-9962498 discloses a spill-resistant semi-solid oral formulation containing a pharmaceutical agent, which can be *inter alia* an analgesic, or a non-steroidal anti-inflammatory drug, such as ibuprofen, and a thickening agent. Preferably, a water-soluble carboxyvinyl polymer, such as Carbopol^{®} 974, is present. Carbopol^{®} is stated to have a surprising taste masking effect for bitter drugs like acetaminophen.

### SUMMARY OF THE INVENTION

The present invention provides a chewable tablet formulation for taste masking a bitter tasting medicament as further specified in the claims below.

The present invention further provides a chewable tablet formulation for ameliorating, preferably substantially preventing, the throat catch caused by medicaments, particularly ibuprofen.

Advantageously, taste masking of bitter medicaments and amelioration of throat catch is coupled with quick release of the medicaments.

### DETAILED DESCRIPTION OF THE INVENTION

While not wishing to be bound by any particular theory as to why the present invention works, it may wall be that the masking components of the instant composition preferentially bind to the sites in the throat where the throat catch causing active would bind and otherwise cause the unpleasant after-buming sensation referred to herein as "throat catch". Throat catch is in essence a throat burn or tingle rather than a sensation of bitterness. As noted earlier, ibuprofen is a medicament that demonstrates this unpleasant effect when incorporated in a chewable tablet composition and tablets prepared therefrom are subsequently chewed

Another possible way in which the present invention may mask bitter taste and/or prevent throat catch is by coating the throat so that when one chews a tablet containing a medicament that is bitter and/or that causes throat catch, the coating acts to prevent contact of the mouth and throat mucosa with the otherwise bitter and/or throat catch producing agent.

Ibuprofen is a proprionic acid derivative which when incorporated in a chewable tablet and the tablet is chewed causes a delayed, strong, burning sensation in the back of the throat. This effect is hereinafter referred to as "throat catch".

It is extremely difficult to mask the unpleasant taste of a bitter medicament component of a chewable tablet without adversely simultaneously effecting the release rate of the medicament from tablet.

It is even more difficult to minimize the throat catch caused by certain medicaments such as ibuprofen when contained in chewable tablets without simultaneously adversely affecting the release rate of the medicaments from the tablets.

As used herein, quick release is synonymous with immediate release as defined in USP 24 / NF 19, Page 856, which specifies that not less than 80% (Q) of the labeled amount of C₁₃H₁₈O₂ is dissolves in 60 minutes.

The present invention provides a chewable tablet formulation that masks the taste of the bitter active incorporated therein as specified in the claims. Surprisingly and unexpectedly an active that causes throat catch, such as for example, ibuprofen, can be incorporated in the formulation of the instant invention and tablets prepared therefrom exhibit little or no throat catch when chewed.

The following examples are offered to illustrate compositions prepared in accordance with the present invention.

### Examples 1 - 4

| Chewable Ibuprofen Tablet | | | | |
|---|---|---|---|---|
| Ingredients | Milligrams per Tablet | | | |
| | Ex. 1 | Ex.2 | Ex.3 | Ex.4 |
| Micromask® Ibuprofen 70%* | 142.857 | 142.857 | 142.857 | 142.857 |
| Mannitol 35 | 74.426 | 74.426 | 74.426 | 74.426 |
| Mannitol - Pearlitol® SD200 | 318.647 | 152.460 | 152.460 | 152.460 |
| Microcrystalline cellulose | 51.220 | 51.220 | 51.220 | 51.220 |
| Aspartame | 20.000 | 20.000 | 20.000 | 20.000 |
| Sodium starch glycolate | 14.000 | 14.000 | 14.000 | 14.000 |
| Citric acid (anhydrous powder) | 9.600 | 9.600 | 9.600 | 9.600 |
| Carbomer 934P | 3.500 | 5.250 | 14.000 | 35.000 |
| Glycine USP | 30.000 | 30.000 | 30.000 | 30.000 |
| Hydrated silica | 14.000 | 14.000 | 14.000 | 14.000 |
| Talc USP | 14.000 | 14.000 | 14.000 | 14.000 |
| Magnesium stearate | 3.500 | 3.500 | 3.500 | 3.500 |
| Flavor | 2.500 | 2.500 | 2.500 | 2.500 |
| Color | 1.750 | 1.750 | 1.750 | 1.750 |

| | | | | |
|---|---|---|---|---|
| *It should be noted that 142.857 mg of Micromask® Ibuprofen 70%(Particle Dynamics) is equivalent to 100 mg of ibuprofen. To guaranty that the tablet will meet the label claim over time an overage of about 43% was employed. | | | | |

The above compositions, scaled up for the production of 20,000 700 mg tablets, were prepared and tablets compressed therefrom in accordance with the following procedure

The hydrated silica, Carbomer 934P and color are screened through a 40-mesh screen. The Mannitol 35, microcrystalline cellulose, aspartame, sodium starch glycolate, flavor, citric acid, glycine and talc are placed in an appropriately sized twin shell blender. The screened mixture of hydrated silica, Carbomer 934P and color is then added to the twin shell blender and the entire mixture is blended for five minutes. The blended mixture is then screened through a 30-mesh screen then returned to the twin shell blender. The Micromask® Ibuprofen 70 and Mannitol (Pearlitol® SD200 is sifted through a #8 mesh screen. The resultant mix is transferred to the twin shell blender and the entire mix is blended for 10 minutes. The magnesium stearate is sifted through a #40 mesh screen then added to the mixture in the twin shell blender and the resultant mix is subjected to blending for an additional 5 minutes. The resultant final tablet mixture is compressed into tablet cores to an in-process hardness resulting in a target of 10-Sc (range 8-13) with no individual tablet being above 16 Sc.

Tablets are prepared using standard tooling on a standard tablet press.

Tablets prepared from the compositions of Examples 1, 2, 3 and 4 (respectively containing 0.5%, 0.75%, 2% and 5% Carbomer 934) had satisfactory disintegration rates and upon dissolution the ibuprofen active was rapidly released. When chewed the tablets evidenced no bitter taste of the ibuprofen component. More importantly, they evidenced no throat catch.

### Examples 5 - 7

| Chewable Ibuprofen Tablet | | | |
|---|---|---|---|
| Ingredients | Milligrams per Tablet | | |
| | Ex. 5 | Ex. 6 | Ex.7 |
| Micromask® Ibuprofen 70%* | 142.857 | 142.857 | 142.857 |
| Mannitol 35 powder | 74.426 | 74.426 | 74.426 |
| Mannitol - Pearlitol® SD200 | 152.460 | 152.460 | 152.460 |
| Microcrystalline cellulose | 51.220 | 51.220 | 51.220 |
| Emdex®, Dextrates hydrated | 166.187 | 155.687 | 134.687 |
| (Penwest) | | | |
| Aspartame | 20.000 | 20.000 | 20.000 |
| Crospovidone | 14.000 | 14.000 | 14.000 |
| Citric acid (anhydrous powder) | 9.600 | 9.600 | 9.600 |
| Carbomer 971P | 3.500 | 14.000 | 35.000 |
| Glycine USP | 30.000 | 30.000 | 30.000 |
| Hydrated silica | 14.000 | 14.000 | 14.000 |
| Talc USP | 14.000 | 14.000 | 14.000 |
| Magnesium stearate | 3.500 | 3.500 | 3.500 |
| Flavor | 2.500 | 2.500 | 2.500 |
| Color | 1.750 | 1.750 | 1.750 |

| | | | |
|---|---|---|---|
| *It should be noted that 142.857 mg of Micromask® Ibuprofen 70%(Particle Dynamics) is equivalent to 100 mg of ibuprofen. To guaranty that the tablet will meet the label claim over time, an overage of about 43% was employed. | | | |

The above compositions, scaled up for the production of 20,000 700mg tablets, were prepared and tablets compressed therefrom in accordance with the procedure described with regard to Examples 1-4 above.

Tablets prepared from the compositions of Examples 5, 6 and 7 (respectively containing 0.5%, 2% and 5% Carbomer 971P) had satisfactory disintegration rates and upon dissolution the ibuprofen active was rapidly released. When chewed the tablets evidenced no bitter taste of the ibuprofen component. More importantly, they evidenced no throat catch.

**Examples 8 - 10**

| Chewable Ibuprofen Tablet | | | |
|---|---|---|---|
| Ingredients | Milligrams per Tablet | | |
| | Ex.8 | Ex.9 | Ex.10 |
| Micmmask® Ibuprofen 70%* | 142.857 | 142.857 | 142.857 |
| Mannitol powder | 74.426 | 74.426 | 74.426 |
| Mannitol - Pearlitol® SD200 | 152.460 | 152.460 | 152.460 |
| Microcrystalline cellulose | 51.220 | 51.220 | 51.220 |
| Emdex®, Dextrates hydrated | 166.187 | 155.687 | 134.687 |
| (Penwest) | | | |
| Aspartame | 20.000 | 20.000 | 20.000 |
| Crospovidone | 14.000 | 14.000 | 14.000 |
| Citric acid (anhydrous powder) | 9.600 | 9.600 | 9.600 |
| Carbomer 974P | 3.500 | 14.000 | 35.000 |
| Glycine USP | 30.000 | 30.000 | 30.000 |
| Hydrated silica | 14.000 | 14.000 | 14.000 |
| Talc USP | 14.000 | 14.000 | 14.000 |
| Magnesium stearate | 3.500 | 3.500 | 3.500 |
| Flavor | 2.500 | 2.500 | 2.500 |
| Color | 1.750 | 1.750 | 1.750 |

| | | | |
|---|---|---|---|
| *It should be noted that 142.857 mg of Micromask® Ibuprofen 70%(Particle Dynamics) is equivalent to 100 mg of ibuprofen. To guaranty that the tablet will meet the label claim over time an overage of about 43% was employed. | | | |

The above compositions, scaled up for the production of 20,000 700mg tablets, were prepared and tablets compressed therefrom in accordance with the procedure described with regard to Examples 1-4 above.

Tablets prepared from the compositions of Examples 8, 9 and 10 (respectively containing 0.5%, 2% and 5% Carbomer 974P) had satisfactory disintegration rates and upon dissolution the ibuprofen active was rapidly released. When chewed the tablets evidenced no bitter taste of the ibuprofen component. More importantly, they evidenced no throat catch.

### Examples 11 - 13

| Chewable Ibuprofen Tablet | | | |
|---|---|---|---|
| Ingredients | Milligrams per Tablet | | |
| | Ex. 11 | Ex. 12 | Ex.13 |
| Micromask® Ibuprofen 70%* | 142.857 | 142.857 | 142.857 |
| Mannitol powder | 74.426 | 74.426 | 74.426 |
| Mannitol - Pearlitol® SD200 | 152.460 | 152.460 | 152.460 |
| Microcrystalline cellulose | 51.220 | 51.220 | 51.220 |
| Emdex®, Dextrates hydrated | 164.437 | 155.687 | 134.687 |
| (Penwest) | | | |
| Aspartame | 20.000 | 20.000 | 20.000 |
| Crospovidone | 14.000 | 14.000 | 14.000 |
| Citric acid (anhydrous powder) | 9.600 | 9.600 | 9.600 |
| PEG-5M (Sentry® Polyox® | | | |
| WSR N80 NF) | 5.250 | 14.000 | 35.000 |
| Glycine USP | 30.000 | 30.000 | 30.000 |
| Hydrated silica | 14.000 | 14.000 | 14.000 |
| Talc USP | 14.000 | 14.000 | 14.000 |
| Magnesium stearate | 3.500 | 3.500 | 3.500 |
| Flavor | 2.500 | 2.500 | 2.500 |
| Color | 1.750 | 1.750 | 1.750 |

| | | | |
|---|---|---|---|
| *It should be noted that 142.857 mg of Micromask® Ibuprofen 70%(Particle Dynamics) is equivalent to 100 mg of ibuprofen. To guaranty that the tablet will meet the label claim over time, an overage of about 43% was employed. | | | |

The above compositions, scaled up for the production of 20,000 700mg tablets, were prepared and tablets compressed therefrom in accordance with the procedure described with regard to Examples 1-4 above.

Tablets prepared from the compositions of Examples 11, 12 and 13 (respectively containing 0.75%, 2% and 5% PEG-5M) had satisfactory disintegration rates and upon dissolution the ibuprofen active was rapidly released.

When chewed the tablets evidenced no bitter taste of the ibuprofen component. More importantly, they evidenced no throat catch.

### Example 14

| Chewable Ibuprofen Tablet | |
|---|---|
| Ingredients | Milligrams per Tablet |
| | Ex. 14 |
| Micromask® Ibuprofen 70%* | 142.857 |
| Mannitol powder | 74.426 |
| Mannitol - Pearlitol® SD200 | 152.460 |
| Microcrystalline cellulose | 51.220 |
| Emdex®, Dextrates hydrated | 168.487 |
| (Penwest) | |
| Aspartame | 20.000 |
| Crospovidone | 14.000 |
| Citric acid (anhydrous powder) | 9.600 |
| Carbomer 934P | 1.750 |
| PEG-5M (Sentry® Polyox® WSR | |
| N80 NF) | 1.750 |
| Glycine USP | 30.000 |
| Hydrated silica | 14.000 |
| Talc USP | 14.000 |
| Magnesium stearate | 3.500 |
| | |
| Flavor | 2.500 |
| Color | 1.750 |

| | |
|---|---|
| *It should be noted that 142.857 mg of Micromask® Ibuprofen 70%(Particle Dynamics) is equivalent to 100 mg of ibuprofen. To guaranty that the tablet will meet the label claim over time, an overage of about 43% was employed. | |

The above compositions, scaled up for the production of 500 700mg tablets, were prepared and tablets compressed therefrom in accordance with the procedure described with regard to Examples 1-4 above.

Tablets prepared from this composition (containing 0.25% Carbomer 934P and 0.25% PEG-5M), had satisfactory disintegration rates and upon dissolution the ibuprofen active was rapidly released. When chewed the tablets evidenced no bitter taste of the ibuprofen component. More importantly, they evidenced no throat catch.

Examples 15 and 16, which follow, demonstrate that uncoated bitter and/or throat-catch causing medicaments, such as ibuprofen, can be used in the composition of the present invention.

### Examples 15 -16

| Chewable Ibuprofen Tablet | | |
|---|---|---|
| Ingredients | Milligrams per Tablet | |
| | Ex. 15 | Ex. 16 |
| Ibuprofen-50 (uncoated, BASF) | 100.000 | 100.000 |
| Mannitol powder | 74.426 | 74.426 |
| Mannitol - Pearlitol® SD200 | 152.460 | 152.460 |
| Microcrystalline cellulose | 51.220 | 51.220 |
| Emdex®, Dextrates hydrated | 209.044 | 205.544 |
| (Penwest) | | |
| Aspartame | 20.000 | 20.000 |
| Crospovidone | 14.000 | 14.000 |
| Citric acid (anhydrous powder) | 9.600 | 9.600 |
| Carbomer 934P | 3.500 | 7.000 |
| Glycine USP | 30.000 | 30.000 |
| Hydrated silica | 14.000 | 14.000 |
| Talc USP | 14.000 | 14.000 |
| Magnesium stearate | 3.500 | 3.500 |
| Flavor | 2.500 | 2.500 |
| Color | 1.750 | 1.750 |

The above compositions, scaled up for the production of 500 700mg tablets, were prepared and tablets compressed therefrom in accordance with the procedure described with regard to Examples 1-4 above.

Tablets prepared from the compositions of Examples 15 and 16 (respectively containing 0.5% and 1% Carbomer 934P and uncoated ibuprofen) had satisfactory disintegration rates and upon dissolution the ibuprofen active was rapidly released. When chewed the tablets evidenced no bitter taste of the ibuprofen component. More importantly, they evidenced no throat catch.

Compositions of the present invention can include sweetening agents such as sucrose, aspartame, glycine, sodium saccharin or mixtures thereof.

Fillers and flow promoting materials, such as silicon dioxide, can also be included.

Uncoated ibuprofen can also be employed; however, the bitter taste and throat catch caused by ibuprofen, though ameliorated and quite acceptable, may not be fully overcome. Thus, the use of a coated ibuprofen is preferred. The coating should be selected bearing in mind that the goal is to secure taste masking of a medicament coupled with quick release of the medicament.

The taste-masking agent employed in the instant invention is selected from the group consisting of Carbomer 934, Carbomer 971, Carbomer 974, PEG-5M and mixtures thereof.

Preferably, the taste-masking agent is selected from the group consisting of Carbomer 934, Carbomer 974, PEG-5M and mixtures thereof.

More preferably, the taste-masking agent is selected from the group consisting of Carbomer 934, Carbomer 974 and mixtures thereof

Most preferably, the taste-masking agent is Carbomer 934.

Carbomer 934, Carbomer 971 and Carbomer 974 are, respectively, available from B.F. Goodrich as Carbopol®934P NF, Carbopol®971P and Carbopol®974P NF.

PEG-5M is commercially available from Union Carbide Corporation as Sentry® Polyox® WSR N80 NF. It is also known as PEG-5000, meaning a polyethylene glycol wherein m has an average value of about 5000.

The taste-masking agent is employed in an amount sufficient to mask the bitte taste and/or throat catch that would otherwise occur when a like composition but not containing the taste-masking agent, contacts the mouth or throat mucosa.

Generally, the taste-masking agent is present in the composition in an amount from 0.25% to 5.0% by weight, based on the total weight of the composition.

Preferably, the taste-masking agent is present in the composition in an amount from 0.5% to 3.0% by weight, based on the total weight of the composition.

More preferably, the taste-masking agent is present in an amount from 0.5% to 1% by weight, based on the total weight of the composition.

Most preferably, the taste-masking agent is present in an amount of 0.5% by weight, based on the total weight of the composition.

It is important to note that the present invention may be applicable to any dosage form that contains a bitter tasting and/or throat catch-causing medicament, such as ibuprofen, and that contacts the oral or throat mucosa.

A unit dose of the composition of the present invention is in the form of a chewable table. Also disclosed are a quick melt tablet, chewable or quick melt wafer, lozenge, troche, powder, chewing gum or a liquid (i.e. solution, suspension, pediatric drop, nose drop, throat spray, gargle or emulsion).

## Claims

1. A composition in the form of a chewable tablet containing a non-steroidal anti-inflammatory agent that has a bitter taste and/or causes throat catch when in contact with mouth or throat mucosa, **characterized in that** the composition is a mixture comprising
a said non-steroidal anti-inflammatory agent selected from the group consisting of ibuprofen, naproxen and ketoprofen, and
a taste-masking agent selected from the group consisting of Carbomer 934, Carbomer 971, Carbomer 974, PEG-5M and mixtures thereof, in an amount from 0.25% to 5.0% by weight, based on the total weight of the composition.

2. The composition, as claimed in Claim 1, wherein the nonsteroidal anti-inflammatory agent is ibuprofen.

3. The composition, as claimed in Claim 1 or Claim 2, wherein the taste-masking agent is present in an amount from 0.5% to 3.0% by weight, based on the total weight of the composition.

4. The composition, as claimed in any one of Claims 1-3, wherein the taste-masking agent is present in an amount of from 0.5% to 1 %, by weight, based on the total weight of the composition.

5. The composition, as claimed in any one of Claims 1-4, wherein the taste-masking agent is selected from the group consisting of Carbomer 934, Carbomer 974 and mixtures thereof.

6. The composition, as claimed in any one of Claims 1-4, wherein the taste-masking agent is Carbomer 934.

7. The composition, as claimed in any one of Claims 1-6, wherein the taste-masking agent is present in the composition in an amount of 0.5% by weight, based on the total weight of the composition, wherein the bitter non-steroidal anti-inflammatory agent is ibuprofen and wherein the latter is present in an amount of 100 milligram.

8. Use of a composition in the form of a chewable tablet containing ibuprofen and a taste-masking agent selected from the group consisting of Carbomer 934, Carbomer 971, Carbomer 974, PEG-5M and mixtures thereof for the manufacture of a medicament for reducing throat catch caused by contacting throat mucosa with ibuprofen.

9. The use, as claimed in Claim 8, wherein the taste-masking agent is present in the composition in an amount from 0-25% to 5.0% by weight, based on the total weight of the composition.

## Patentansprüche

1. Zusammensetzung in der Form einer kaubaren Tablette, die ein nicht-steroides, entzündungshemmendes Mittel enthält, das einen bitteren Geschmack aufweist und/oder Rachenreizung verursacht, wenn es mit Mund- oder Rachenschleimhaut in Kontakt kommt, **dadurch gekennzeichnet, dass** die Zusammensetzung ein Gemisch darstellt, umfassend
ein nicht-steroides, entzündungshemmendes Mittel, ausgewählt aus der Gruppe, bestehend aus Ibuprofen, Naproxen und Ketoprofen, und
ein Geschmack maskierendes Mittel, ausgewählt aus der Gruppe, bestehend aus Carbomer 934, Carbomer 971, Carbomer 974, PEG-5M und Gemischen davon, in einer Menge von 0,25 % bis 5,0 Gew.-%, bezogen auf das Gesamtgewicht der Zusammensetzung.

2. Zusammensetzung nach Anspruch 1, worin das nicht-steroide, entzündungshemmende Mittel Ibuprofen darstellt.

3. Zusammensetzung nach Anspruch 1 oder Anspruch 2, worin das den Geschmack maskierende Mittel in einer Menge von 0,5 % bis 3,0 Gew.-%, bezogen auf das Gesamtgewicht der Zusammensetzung, vorliegt.

4. Zusammensetzung nach einem der Ansprüche 1-3, worin das den Geschmack maskierende Mittel in einer Menge von 0,5 % bis 1 Gew.-%, bezogen auf das Gesamtgewicht der Zusammensetzung, vorliegt.

5. Zusammensetzung nach einem der Ansprüche 1-4, worin das den Geschmack maskierende Mittel aus der Gruppe, bestehend aus Carbomer 934, Carbomer 974 und Gemischen davon, ausgewählt ist.

6. Zusammensetzung nach einem der Ansprüche 1-4, worin das den Geschmack maskierende Mittel Carbomer 934 darstellt.

7. Zusammensetzung nach einem der Ansprüche 1-6, worin das den Geschmack maskierende Mittel in der Zusammensetzung in einer Menge von 0,5 Gew.-%, bezogen auf das Gesamtgewicht der Zusammensetzung, vorliegt, wobei das bittere, nicht-steroide, entzündungshemmende Mittel Ibuprofen darstellt und wobei das Letztere in einer Menge von 100 Milligramm vorliegt.

8. Verwendung einer Zusammensetzung in Form einer kaubaren Tablette, die Ibuprofen und ein den Geschmack maskierendes Mittel, ausgewählt aus der Gruppe, bestehend aus Carbomer 934, Carbomer 971, Carbomer 974, PEG-5M und Gemischen davon, enthält, zur Herstellung eines Medikaments für das Vermindern von Rachenreizung, die durch In-Kontakt-Kommen der Rachenschleimhaut mit Ibuprofen verursacht wird.

9. Verwendung nach Anspruch 8, worin das den Geschmack maskierende Mittel in der Zusammensetzung in einer Menge von 0,25 % bis 5,0 Gew.-%, bezogen auf das Gesamtgewicht der Zusammensetzung, vorliegt.

## Revendications

1. Composition sous forme d'un comprimé à mâcher contenant un agent anti-inflammatoire non-stéroïdien qui a un goût amer et/ou qui provoque une irritation de la gorge ou une muqueuse de la gorge, **caractérisée en ce que** la composition est un mélange comprenant
ledit agent anti-inflammatoire non-stéroïdien sélectionné à partir du groupe composé d'ibuprofène, naproxène et kétoprofène, et
un agent masquant le goût sélectionné à partir du groupe composé de Carbomère 934, Carbomère 971, Carbomère 974, PEG-5M et des mélanges de ceux-ci, selon une quantité comprise dans la gamme allant de 0,25% à 5,0% en poids, sur base du poids total de la composition.

2. Composition selon la revendication 1, **caractérisée en ce que** l'agent anti-inflammatoire non-stéroïdien est de l'ibuprofène.

3. Composition selon la revendication 1 ou 2, **caractérisée en ce que** l'agent masquant le goût est présent selon une quantité comprise dans la gamme allant de 0,5% à 3,0% en poids, sur base du poids total de la composition.

4. Composition selon l'une quelconque des revendications 1-3, **caractérisée en ce que** l'agent masquant le goût est présent selon une quantité comprise dans la gamme allant de 0,5% à 1%, en poids, sur base du poids total de la composition.

5. Composition selon l'une quelconque des revendications 1-4, **caractérisée en ce que** l'agent masquant le goût est sélectionné à partir du groupe composé de Carbomère 934, Carbomère 974 et des mélanges de ceux-ci.

6. Composition selon l'une quelconque des revendications 1-4, **caractérisée en ce que** l'agent masquant le goût est du Carbomère 934.

7. Composition selon l'une quelconque des revendications 1-6, **caractérisée en ce que** l'agent masquant le goût est présent dans la composition selon une quantité comprise dans la gamme allant de 0,5% en poids, sur base du poids total de la composition, **caractérisée en ce que** l'agent anti-inflammatoire non-stéroïdien amer est de l'ibuprofène et **caractérisée en ce que** ce dernier est présent selon une quantité de 100 milligrammes.

8. Utilisation d'une composition sous forme d'un comprimé à mâcher contenant de l'ibuproféne et un agent masquant le goût sélectionné à partir du groupe composé de Carbomère 934, Carbomère 971, Carbomère 974, PEG-5M, et des mélanges de ceux-ci, dans la fabrication d'un médicament destiné à réduire l'irritation de la gorge qui est provoquée lors de la mise en contact des muqueuses de la gorge avec de l'ibuprofène.

9. Utilisation selon la revendication 8, **caractérisée en ce que** l'agent masquant le goût est présent dans la composition selon une quantité comprise dans la gamme allant de 0,25% à 5,0% en poids, sur base du poids total de la composition.
